(19) Europäisches Patentamt
European Patent Office
Office européen des brevets

(11) **EP 2 290 370 B1**

(12) **EUROPEAN PATENT SPECIFICATION**

(45) Date of publication and mention
of the grant of the patent:
**03.12.2014 Bulletin 2014/49**

(21) Application number: **09766412.2**

(22) Date of filing: **16.06.2009**

(51) Int Cl.:
**G01N 33/86** (2006.01)

(86) International application number:
**PCT/JP2009/002721**

(87) International publication number:
**WO 2009/153964 (23.12.2009 Gazette 2009/52)**

(54) **METHOD FOR DETERMINING CAUSE OF THE PROLONGATION OF BLOOD COAGULATION TIME**

VERFAHREN ZUR BESTIMMUNG DES FAKTORS, DER DIE VERLÄNGERUNG DER BLUTGERINNUNGSZEIT VERURSACHT

PROCÉDÉ PERMETTANT DE DÉTERMINER UN FACTEUR RESPONSABLE DE LA PROLONGATION DU TEMPS DE COAGULATION SANGUINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **18.06.2008 JP 2008159144**

(43) Date of publication of application:
**02.03.2011 Bulletin 2011/09**

(73) Proprietor: **Sekisui Medical Co., Ltd.**
**Tokyo 103-0027 (JP)**

(72) Inventors:
• **NAKAMURA, Remi**
**Ryugasaki-shi,**
**Ibaraki 301-0852 (JP)**
• **MORIKAWA, Chizuru**
**Ryugasaki-shi,**
**Ibaraki 301-0852 (JP)**
• **SUNAGA, Hiroyuki**
**Tokyo 103-0027 (JP)**
• **YAGO, Hirokazu**
**Ryugasaki-shi,**
**Ibaraki 301-0852 (JP)**

(74) Representative: **Hartz, Nikolai**
**Wächtershäuser & Hartz**
**Patentanwaltspartnerschaft mbB**
**Weinstrasse 8**
**80333 München (DE)**

(56) References cited:
**JP-A- 10 104 239        JP-A- 2004 069 697**
**JP-A- 2006 349 684        JP-T- 2002 519 635**

• **JACOBSEN, E. M. ET AL.: 'Detection and quantification of lupus anticoagulants in plasma from heparin treated patients, using addition of polybrene' THROMB J vol. 4, no. 3, 2006, XP021009570**
• **LIESTOL, S. ET AL.: 'Dilute prothrombin time-based lupus ratio test. Integrated LA testing with recombinant tissue thromboplastin' THROMB RES 2002 vol. 105, 2002, pages 177 - 182, XP002485145**
• **MASAHIRO IEKO: 'Ko-Rinshishitsu Kotai Shokogun ni Okeru Rinsho Kensa no Atarashii Tenkai -Mixing Test no Yuyosei' JAPANESE JOURNAL OF THROMBOSIS AND HEMOSTASIS vol. 19, no. 5, 01 October 2008, page 640, XP008147015**
• **KEN ARAI ET AL.: 'Zenjido Ketsueki Gyoko Bunseki Sochi Coapresta 2000 ni yoru Mixing Test no Hyoka' THE JAPANESE JOURNAL OF CLINICAL PATHOLOGY vol. 56, no. SUPP., 31 October 2008, page 172**
• **SUMIYOSHI NAITO ET AL.: 'Zenjido Ketsueki Gyoko Bunseki Sochi Coapresta 2000 o Mochiita Kongo Hosei Shiken no Kento' THE JAPANESE JOURNAL OF CLINICAL PATHOLOGY vol. 56, no. SUPP., 31 October 2008, page 171**

EP 2 290 370 B1

**Description**

Technical Field

The present invention relates to a method for determining a cause of the prolongation of blood coagulation time in a blood sample.

Background Art

A blood coagulation test is carried out for screening of the presence or absence of any abnormality in the blood coagulation reaction system, or for measurement of the activity of individual factors, by measuring the time period starting from the time point at which an activator and/or $Ca^{2+}$ and the like are added into a specimen or a specimen mixture, until the time point at which detectable fibrin clots are formed (blood coagulation time; hereinafter, occasionally also referred to simply as coagulation time). Typical examples of the blood coagulation time include prothrombin time (PT), activated partial thromboplastin time (APTT), and thrombin time.

The PT is the time taken from the addition of a mixed liquid of tissue thromboplastin and $Ca^{2+}$ to a test plasma until the coagulation of the blood plasma, and the PT is intended for a comprehensive examination of the coagulation activities of factor VII, factor X, factor V, prothrombin, fibrinogen and the like, which are related to the extrinsic pathway of coagulation. The APTT is the time taken from the addition of a sufficient amount of phospholipids and an activating agent (kaolin, silicic anhydride, ellagic acid, or the like) and an appropriate amount of $ca^{2+}$ to a test plasma until the coagulation of the blood plasma, and the APTT is intended for a comprehensive examination of the coagulation activities of factor XII, factor XI, prekalikrein, high molecular weight kininogen, factor IX, factor VIII, factor X, factor V, prothrombin, fibrinogen and the like, which are related to the intrinsic pathway of coagulation.. In general, what is referred to as abnormality in these blood coagulation tests is the prolongation of the coagulation time. Abnormality in the blood coagulation reaction system reflects the signs of the tendency to hemorrhage or the tendency to thrombosis (tendency to blood coagulation) in the body.

Causes of these abnormalities that can be considered include: 1) a deficiency or decrease in the blood coagulation factors, 2) the presence of an antibody to a blood component that constitutes the blood coagulation system, 3) the presence of an antibody to a component in the reagent for measuring the blood coagulation time, 4) the presence of an antibody to a composite between a blood component that constitutes the blood coagulation system and a component in the reagent for measuring the blood coagulation time, and 5) administration of a drug that inhibits the blood coagulation reaction.

However, simply performing the measurement of the blood coagulation time does not enable differentiation of whether the cause is a decrease in the activity due to simple deficiency of coagulation factors, or a decrease in the activity due to the inhibition of the coagulation reaction by an antibody (inhibitor) to a component or the like in a component that constitutes the blood coagulation system or a component in the reagent for measuring the blood coagulation time. Furthermore, since the therapeutic policy varies with the difference in the causes, differentiation of the cause is important. Thus, there has been carried out a blood coagulation correction test (mixing test) in which normal plasma is added to a test plasma, and the extent of the blood coagulation time of the test plasma being corrected (normalized) is plotted to determine the cause (Non-Patent Document 1).

The mixing test has been traditionally carried out, for example, in the manner described below.

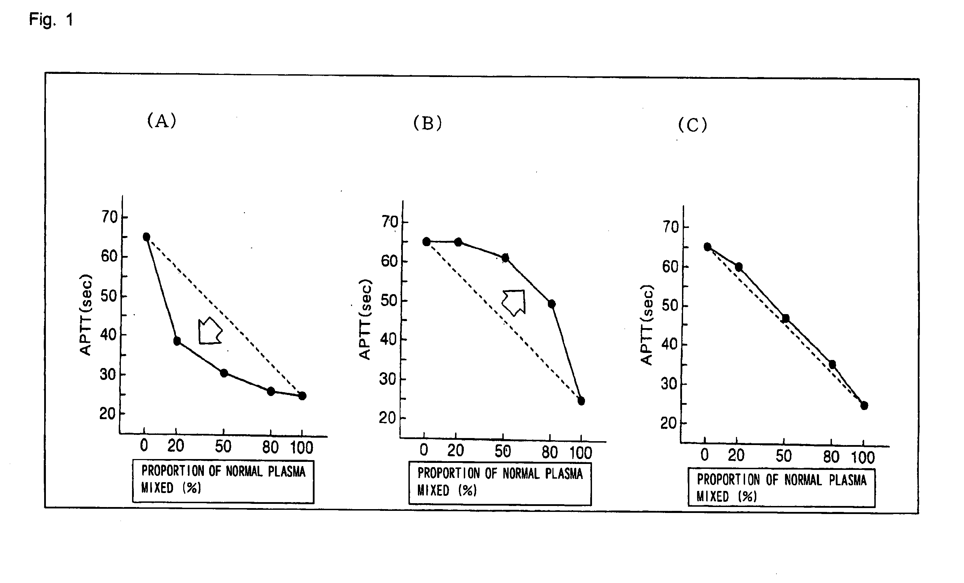

A sample is prepared by adding normal plasma to test plasma and mixing them such that the proportion of the normal plasma is 0, 20, 50, 80 or 100%, and the APTT is measured. The results are plotted into a graph (horizontal axis: proportion of the normal plasma mixed or the proportion of the test plasma (%), vertical axis: coagulation time (seconds)), and the cause is visually determined from the shape of the graph. For example, in the case of a deficiency of a coagulation factor, the addition of a small amount of normal plasma (20% in FIG. 1(A)) significantly shortens the coagulation time to thereby bring the coagulation time close to the value obtainable in the case of normal plasma. Therefore, the graph shows a convex curve below a straight line (dotted line) connecting the points corresponding to the test plasma and the normal plasma (FIG. 1(A)).

When a coagulation factor inhibitor is present, the inhibitor inactivates the coagulation factor in the normal plasma, even though the proportion of the normal plasma added is increased. Therefore, the extent of an improvement in the coagulation time due to the addition of normal plasma is low, and a convex curve is shown above the straight line (FIG. 1(B)).

However, a graph with a shape similar to the dotted line (FIG. 1(C)) may be obtained depending on the specimen, and in that case, there is a problem that it is very difficult to make a determination.

Furthermore, since the mixing test involves making a determination by visual inspection, there is no unified method for quantifying the extent of correction, and the final determination is entrusted to the judge. Therefore, there is another problem that it is possible to have the result of determination varied depending on the degree of proficiency of the judge.

Moreover, there is also a possibility that the result of determination may vary depending on the difference in the sensitivity of the reagent for measurement to the coagulation factor inhibitor. Particularly, lupus anticoagulant (hereinafter, LA) is known as a coagulation factor inhibitor that depends on the sensitivity of the reagent. The LA is not an inhibitor to individual coagulation factors, but is an immunoglobulin that inhibits the phospholipid-dependent coagulation reaction. Since the presence of phospholipids is essential to the coagulation reaction, usually, many of the reagents for measuring blood coagulation are rich in phospholipids. The LA reacts with the phospholipids in the reagent, thereby consuming these phospholipids, and consequently inhibits the coagulation reaction. Therefore, coagulation tests suchas PT and APTT are often found tobe abnormal. However, since the LA results in reaction intensities that vary with the type of the phospholipids (origin, phospholipid composition, and the like), it is known to obtain different results of determination based on the reagent used.

There are also known methods for determination as follows, which do not depend on the degree of proficiency of the judge and facilitate the determination (Non-Patent Document 2). When the coagulation time for a test plasma is designated as a, the coagulation time for a mixture of a test plasma and a normal plasma at a mixing ratio of 5:5 is designated as b, and the coagulation time for a normal plasma is designated as c,

$$1)\ (a+c)/2 \le b, \quad 2)\ (b-c)/a \ge x, \quad 3)\ b-c \ge x, \quad 4)\ b/c \ge x$$

The method for determination (1) merely expresses in numerical values of whether the graph is convex above the straight line, or the graph is convex below the straight line, and thus the determination by visual inspection and the results do not change. The method for determination (2) is also known as Rosner Index, and is considered as a useful method for determination in determining the LA in particular. In this method, a value of 0.15 is considered appropriate for x, but this value is a value set up for the kaolin coagulation time (KCT). Actually, each reagent used in various facilities requires appropriate setting for x, but the methods for such setting are not clearly known (Non-Patent Document 3). The methods for determination (3) and (4) also require setting of x for various facilities, but the methods for such setting are not clearly known, and cannot be said to be satisfactory as methods for determination.

Documents of Related Art

Non-Patent Document

Non-Patent Document 1: Kensa To Gijutsu (Examination and Technology), Vol. 34, no. 8, August 2006, p. 735-742
Non-Patent Document 2: Rinsho Kensaho Teiyo (Compendium of Clinical Examination Methods), 32nd Edition, p. 443
Non-Patent Document 3: Thrombo. Haemost. Vol. 57, no. 2, 1987, p. 144-147

Disclosure of Invention

Problems to be Solved by the Invention

As discussed above, the conventional methods lack clarity in the technique of setting the cut-off value, and have a risk that the coagulation factor inhibitors miss weakly positive specimens. Actually, in the review carried out by the inventors of the present invention, there has been acknowledged an example in which the graph obtained by a mixing test in the case of a LA weakly positive specimen shows a convex curve below the straight line. Furthermore, the mixing test is largely dependent on the difference in the sensitivity to the coagulation factor inhibitor of the reagents for measurement, and there is a possibility that the result of measurement may vary with the reagent. Moreover, the method of determining by visual inspection greatly depends on the experience of the judge as described above, and has a risk that the result of determination may vary with the difference in the level of proficiency.

Therefore, there is a strong demand for the development of a method for determination which does not depend on the intensity of the sensitivity to a coagulation factor inhibitor in a reagent or on the difference in the level of proficiency of the judge, and which is intended to obtain consistent results of determination in regard to whether the prolongation of the blood coagulation time is caused by the presence of a coagulation factor inhibitor or by a deficiency of a coagulation factor. Means for Solving the Problems

The inventors of the present invention made a thorough investigation, and as a result, they found that the problems described above can be solved by a method for determination as shown below, in which the results of a mixing test are not by determined by visual inspection but by a comparison of the area ratio in the graph, and the setting of the cut-off value is also clearly defined. Thus, the inventors completed the present invention.

Specifically, the present invention provides the following inventions.

1. A method of determining a cause of prolongation of blood coagulation time in test plasma, the method including:

(1) measuring blood coagulation times of samples including (a) the test plasma only, (b) normal plasma only, and (c) the test plasma and the normal plasma mixed at least at a mixing ratio;
(2) drawing a polygonal line graph by plotting the measurement results of the samples (a), (b) and (c), with a vertical axis representing the blood coagulation time or a prolongation ratio of blood coagulation time and a horizontal axis representing the mixing ratio or mixing proportion of the test plasma or the normal plasma, and thereby determining an area A under the polygonal line and an area B under a line segment that connects the plotted measurement results of the samples (a) and (b);
(3) calculating a ratio of the area (A-B) obtained by subtracting the area B from the area A, with respect to the area B, ((A-B)/(B)): area ratio X);
(4) performing the steps of (1) and (2) for a coagulation factor inhibitor-positive plasma, and thereby determining in advance a standard area ratio Y; and
(5) comparing the area ratio X and the standard area ratio Y, and determining the test plasma as a coagulation factor inhibitor type in the case where $Y \leq X$, or as a coagulation factor deficient type in the case where $Y > X$.

2. The method of 1, wherein the standard area ratio Y for the coagulation factor inhibitor-positive plasma is a value determined with respect to a coagulation factor inhibitor-positive plasma exhibiting a blood coagulation time that is larger than the upper limit of a standard range which has been obtained by statistically processing the blood coagulation time of a plurality of healthy persons' plasmas, and is not greater than a value of the upper limit + 50%.
3. The method of 2, wherein the standard range for the blood coagulation time of healthy persons' plasmas was obtained by statistically processing the blood coagulation time of a plurality of healthy persons' plasmas.
4. The method of 1, wherein when the vertical axis is used to represent the blood coagulation time in (2) of 1, values obtained by subtracting a constant number therefrom are used for the area A and the area B.
5. The method of 4, wherein the constant number is obtained by drawing an auxiliary line parallel to the horizontal axis in the plot of the blood coagulation time of the normal plasma drawn in the form of a polygonal line graph, and calculating the area under the auxiliary line.
6. The method of any one of 1 to 5, wherein the blood coagulation time is at least one selected from PT (Prothrombin time), APTT (activated partial thromboplastintime), dPT (dilute PT), dAPTT (dilute APTT), KCT (kaolin coagulation time), and dRVVT (dilute Russell's viper venom time).
7. The method of any one of 1 to 6, wherein the coagulation factor inhibitor is at least one selected from Lupus anticoagulant (LA), a factor VIII inhibitor, a factor IX inhibitor, and a factor V inhibitor.

Effects of the Invention

According to the method of the present invention, the determination of plasma in which the coagulation factor inhibitor is weakly positive, for which determination has been traditionally difficult, can be more accurately carried out. Furthermore, according to the method of the present invention, accurate determination can be easily carried out, without being affected by the degree of proficiency of the judge. Therefore, according to the method of the present invention, a proper therapeutic policy for a patient with prolonged coagulation time can be determined by a blood coagulation time test.

Brief Description of Drawings

FIG. 1 is a diagram showing the results of a mixing test according to a conventional method. FIG. 1(A) represents a coagulation factor deficiency type, FIG. 1(B) represents a coagulation factor inhibitor type, and FIG. 1(C) represents a pattern of an undefined cause;
FIG. 2 is a diagram showing an example of the area under the polygonal line graph of step (2);
FIG. 3 is a diagram showing an example of the area under the polygonal line graph of step (2);
FIG. 4 is a diagram showing an example of the area (A-B) of step (3) (corresponding to FIG. 2);
FIG. 5 is a diagram showing an example of the area (A-B) of step (3) (corresponding to FIG. 3);
FIG. 6 is a polygonal line graph plotted for specimen No. 1;

FIG. 7 is a polygonal line graph plotted for specimen No. 4; and
FIG. 8 is a polygonal line graph plotted for specimen No. 6.

Best Mode for Carrying out the Invention

As the test plasma to be used in the present invention, it is preferable to use blood plasma, rather than to use the blood directly, and it is more preferable to use platelet-poor plasma. This is because the platelet-derived phospholipids remaining in the blood plasma can eliminate the possibility of a phospholipid-dependent coagulation factor inhibitor such as LA becoming negative.

Normal blood plasma that is used in the mixing with the test plasma maybe self-prepared or commercially available normal plasma, and preferably, normal plasma which has been subjected to platelet removal is preferable. Known techniques may be used for the method of removing platelets as described above, and for example, centrifugation or platelet removal filter treatment can be used. Known examples of commercially available products include Pooled Normal Plasma (manufactured by Precision Biologic, Inc.), Platelet Poor Plasma (Technoclone GmbH), andLAtrol (Trade Mark) Normal Control (American Diagnostica, Inc.).

Known examples of the coagulation factor inhibitor include, but not limited to, a factor VIII inhibitor, a factor IX inhibitor and a factor V inhibitor, in addition to the LA. The method of the present invention can be applied, when a reagent for measuring the blood coagulation time which shows sensitivity to the subject coagulation factor inhibitor is used.

For example, when the LA is used as the subj ect, any reagent for measuring the time of phospholipid-dependent blood coagulation, which reagent exhibits sensitivity to the LA, may be used, and reagents that measure the PT, APTT, dAPTT, dPT, KCT and dRVVT are in use. Furthermore, when the factor VIII inhibitor or the factor IX inhibitor is used as the subject, an APTT measuring reagent or the like is used, and when the factor V inhibitor is used as the subject, an APTT measuring reagent, a PT measuring reagent or the like is used. Commercially available products can be used for all of these reagents. For instance, examples of commercially available PT measuring reagents include Coagpia (registered trademark) PT-S (manufactured by Sekisui Medical Co., Ltd.), ThromboCheck PT Plus (manufactured by Sysmex Corp.), and STA Reagent Series PT (manufactured by F. Hoffmann-La Roche, Ltd.). Examples of commercially available APTT measuring reagents include Coagpia (registered trademark) APTT-S (manufactured by Sekisui Medical Co.,Ltd.), ThromboCheck APTT-SLA (manufactured by Sysmex Corp.), and APTT Liquid RD (manufactured by F. Hoffmann-La Roche, Ltd.).

On the other hand, examples of a coagulation factor, which is suspected to be deficient or reduced due to an abnormality of the blood coagulation test, include fibrinogen, prothrombin, factor V, factor VII, factor VIII, factor IX, factor X, factor XI, factor XII, prekalikrein, high molecular weight kininogen, and von Willebrand factor (vWF).

In step (1) of the method for determination of the invention, the blood coagulation time of samples including (a) test plasma only, (b) normal plasma only, and (c) a mixed sample obtained as described above, are measured.

In the measurement of the blood coagulation time (mixing test) using a sample prepared by mixing a test plasma and a normal plasma at least at one mixing ratio, the plot number may be 3 points or higher including the test plasma and the normal plasma themselves, but in order to clarify the variation pattern of the coagulation time that accompanies the variation of the mixing ratio between the test plasma and the normal plasma, the plot number is preferably 4 points or higher, and more preferably 5 points or higher. The upper limit of the plot number is not particularly limited, but when the economic efficiency or measurement time is considered, the plot number is preferably 10 points or lower, and more preferably 7 points or lower.

The prolongation ratio of the coagulation time is a relative ratio defined when the difference between the coagulation time of normal plasma and the coagulation time of test plasma only is taken as 1. As a matter of fact, the prolongation ratio may also be represented by the relative percentage defined by taking the difference in the coagulation time as 100.

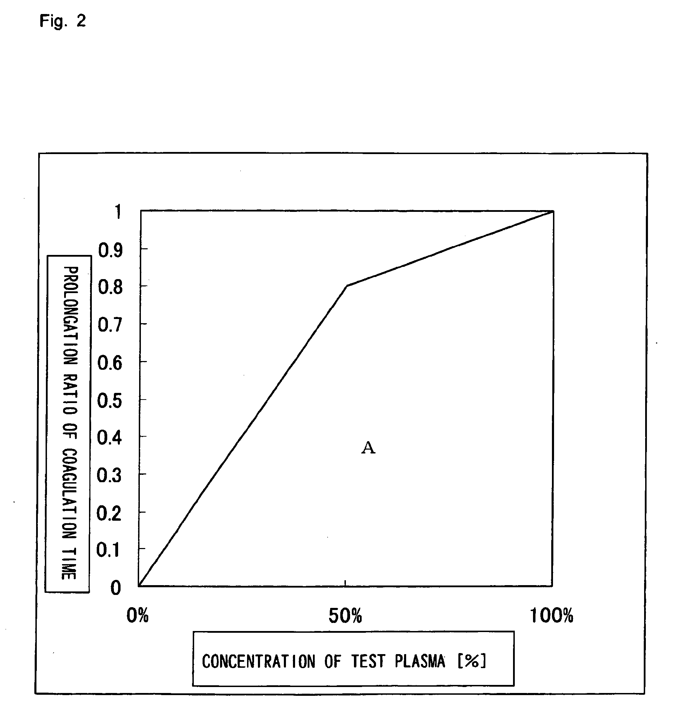

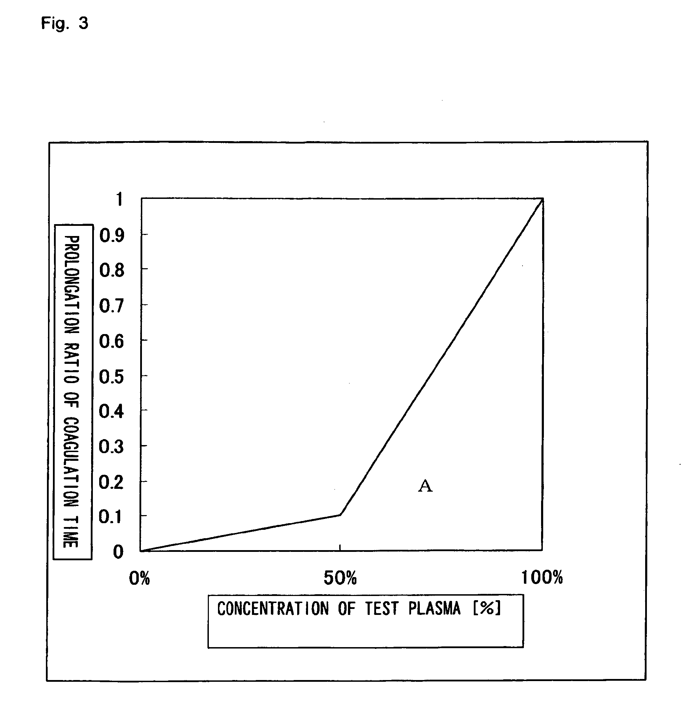

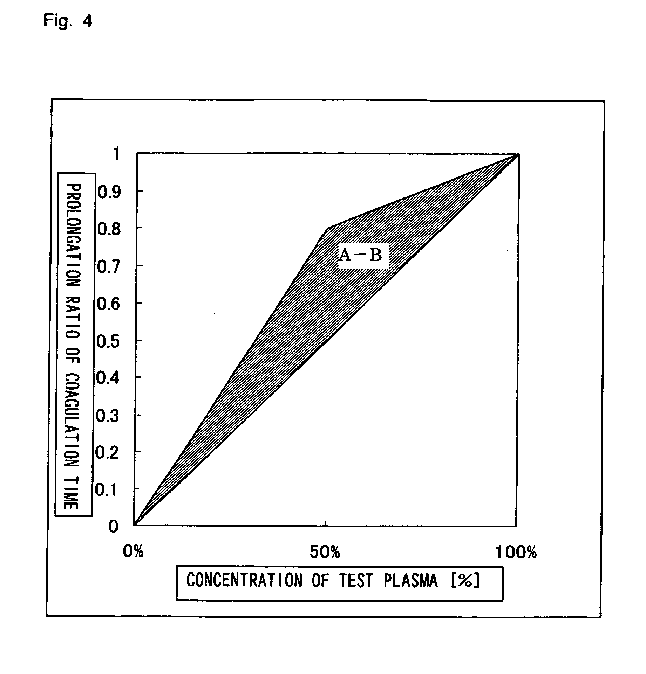

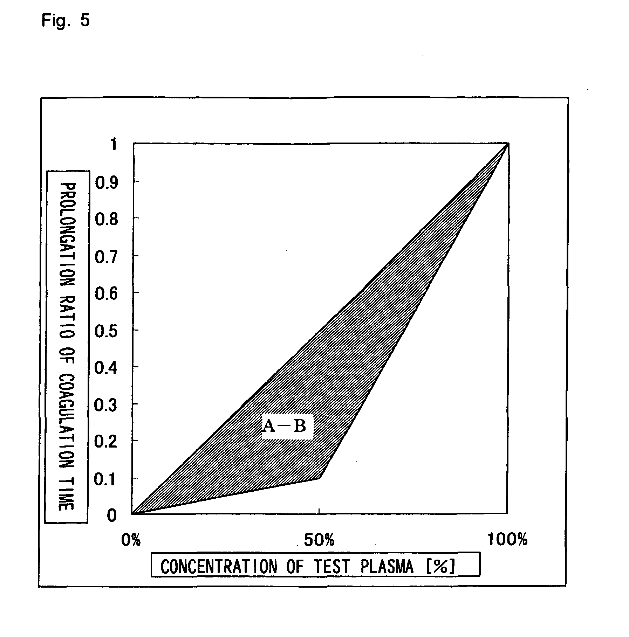

Step (2) is a step carried out by drawing a polygonal line graph using the results of measurement of (a) the coagulation time of the sample containing the test plasma only, (b) the coagulation time of the sample containing a normal plasma only, and (c) the coagulation time of the sample containing a mixed liquid thereof, and plotting the results of measurement of the items (a), (b) and (c), with the vertical axis representing the blood coagulation time or the prolongation ratio of blood coagulation time and the horizontal axis representing the mixing ratio ormixingproportion of the test plasma or the normal plasma, so as to determine the area A under the polygonal line and the area B under a line segment that connects the plotted results of measurement of the items (a) and (b). For example, FIGs. 2 and 3 present the examples of polygonal line graph in the case of taking the vertical axis to represent the prolongation ratio of the coagulation time (plot number 3, that is, an example in which the mixed liquid is of a single kind). Here, the area (A) is the area of, for example, a square shown in FIG. 2 or 3. FIG. 4 is a diagram with a diagonal line added to FIG. 2, and FIG. 5 is a diagram with a diagonal line added to FIG. 3. The area (B) is the area of a right-angled triangle having the diagonal line as the hypotenuse, as shown in FIG. 4 or 5. Furthermore, in the case of taking the vertical axis to represent the blood coagulation time, the values of the area A and area B may be directly used, or values obtainable after subtracting a constant number from both the areas can also be used. As the constant number, for example, when an auxiliary line parallel to the horizontal axis is drawn in a plot of the blood coagulation time of the normal plasma, the value of the area under the auxiliary line can be used.

Step (3) is a step of calculating the ratio of the area (A-B), which is the value obtainable by subtracting the area (B) of a right-angled triangle having the diagonal line connecting the blood coagulation time or the prolongation ratio of the coagulation time of the sample containing the test plasma only and the sample containing the normal plasma only, from the area (A) of a polygon formed by the polygonal line graph, the horizontal axis and the vertical axis, with respect to the area of the right-angled triangle, ((A-B) / (B)) : area ratio X). The area ratio may also be expressed in percentage.

In the case of FIG. 4, since the area of (A) is larger than the area (B), the ratio (A-B)/(B) is a positive number. On the other hand, in the case of FIG. 5, since the area (A) is smaller than the area (B), the ratio (A-B) / (B) is a negative number.

Step (4) is a step of setting the cut-off value.

It is desirable touse, as the sample for setting the cut-off value, a coagulation factor inhibitor-positive plasma exhibiting a coagulation time that is close to the coagulation time for a healthy person's plasma, in order to increase the detection sensitivity of the coagulation factor inhibitor. For example, the blood coagulation time of a sample for setting the cut-off value is larger than the upper limit of a standard range for healthy persons, which has been obtained by statistically processing the blood coagulation time of plural healthy persons' plasmas, and the blood coagulation time of the sample for setting the cut-off value is preferably not greater than the value of the upper limit + 50%, more preferably not greater than the value of the upper limit + 20%, and even more preferably not greater than the value of upper limit + 10%.

Furthermore, any coagulation factor inhibitor-positive plasma falling in the above range maybe used, and weakly positive plasma may be used, or a series of stepwise dilutions of strongly positive plasma in normal plasma may also be used.

Commercially available products can also be used as the known coagulation factor inhibitor-positive plasma mentioned above. Known examples of the commercially available products of LA-positive plasma include LAtrol (Trade Mark) (American Diagnostica, Inc.), Lupus Positive Control (manufactured by Precision Biologic, Inc.), and Human Plasma Lupus Anticoagulant (George King Bio-Medical, Inc.). Known examples of the commercially available products of factor VIII inhibitor include Human Plasma FVIII with Inhibitor (George King Bio-Medical, Inc.) and Factor VIII Inhibitor Plasma (Technoclone GmbH).

However, the cut-off value (standard area ratio Y) may be appropriately set up for each facility with which the mixing test is performed, in consideration of the differences in the reagent used or the measuring instrument.

In addition, an identical product is used for the reagent for measuring the blood coagulation time of a diluted sample and the reagent for measuring the blood coagulation time of a healthy person. As an example, the examples of APTT in the case of using Coagpia (Registered Trademark) APTT-S (manufactured by Sekisui Medical Co., Ltd.; hereinafter, reagent A) and Thrombocheck APTT-SLA (manufactured by Sysmex Corp.; hereinafter, reagent B) are presented in Table 1.

[Table 1]

| APTT | | | |
|---|---|---|---|
| | | | [unit: seconds] |
| | | Reagent A | Reagent B |
| | Standard range | 26.5 to 36.6 | 24.6 to 35.7 |
| Dilution rate | 1/4 | 51.5 | 43.2 |
| | 1/6 | 46.1 | 39.9 |
| | 1/8 | 39.9 | 35.8 |
| | 1/10 | 36.0 | 33.4 |
| | 1/12 | 35.5 | 32.8 |
| | 1/14 | 34.3 | 31.5 |
| | 1/16 | 33.9 | 30.7 |

In the Table 1, in the case of the reagent A, the 8-fold diluted sample exhibiting an APTT value of 39.9 seconds, which is larger than the standard range of healthypersons, 36.6 seconds, serves as the sample for setting the cut-off value. In the case of the reagent B, the 8-fold diluted sample exhibiting an APTT value of 35.8 seconds, which is larger than the standard range of healthy persons, 35.7 seconds, serves as the sample for setting the cut-off value. Here, the standard range of healthy persons is preferably obtained by statistically processing the blood coagulation time of plural healthy persons' plasmas, as will be described below.

Step (5) is a step of determining whether the test plasma is of the coagulation factor inhibitor type or of the coagulation factor deficient type. The area ratio X is compared with the standard area ratio Y, and in the case where Y $\leq$ X, the test plasma can be determined as the coagulation factor inhibitor type, while in the case where Y > X, the test plasma can be determined as the coagulation factor deficient type. Since the determination is based on a comparison of numerical values, the determination is clear and does not require proficiency. Here, the coagulation factor inhibitor type implies that the prolongation of the blood coagulation time is caused by the coagulation factor inhibitors discussed above. The coagulation factor deficient type implies that the prolongation of the blood coagulation time is caused by the deficiency of the coagulation factors.

The area or area ratio as discussed above maybe calculated by actually plotting a graph, or the like, but as long as the same calculation results are obtained, the method of calculating the area or area ratio is not limited. That is, the instrument for measuring the coagulation time maybe imparted with a function such as one capable of obtaining the relevant calculation results, so that the calculation is automatically carried out from the results of sample analysis.

Examples

Hereinafter, the present invention will be described in more detail by way of Examples, but the present invention is not intended to be limited to these.

Example 1

(1) Determination of sample for setting cut-off value

50 $\mu$L each of specimens prepared by diluting a LA-positive plasma with a normal plasma to 4 to 16 times were provided, and 50 $\mu$L each of a reagent for measuring APTT was added thereto. The mixtures were heated to 37°C for 3 minutes, and then 50 $\mu$L each of a calcium chloride liquid was added to the mixtures. The coagulation time was measured using a fully automated blood coagulation analyzer, Coapresta (registered trademark) 2000 (sold by Sekisui Medical Co., Ltd.).

Lupus Positive Control was used as the LA-positive plasma, and Pooled Normal Plasma was used as the normal plasma (all manufactured by Precision Biologic, Inc.). Furthermore, Coagpia (registered trademark) APTT-S (manufactured by Sekisui Medical Co., Ltd.; hereinafter, reagent A) and ThromboCheck APTT-SLA (manufactured by Sysmex Corp.; hereinafter, reagent B) were used as the reagents for measuring the APTT. The normal standard ranges of the reagents were set up based on the measurement values measured in 48 healthy persons by using each of the reagents, and were set as the mean value $\pm$ 2SD of the coagulation time.

As shown in the Table 1, in the case of the reagent A, since the 10-fold diluted specimen resulted in an APTT value of 36.0 seconds, which was within the normal standard range (26.5 to 36.6 seconds), itwasdecidedtousethe8-folddilutedspecimen for the setting of the cut-off value. Likewise, in the case of the reagent B, since the 10-fold diluted specimen resulted in an APTT value of 33.4 seconds, which was within the normal standard range (24.6 to 35.7 seconds), it was decided to use the 8-fold diluted specimen for the setting of the cut-off value.

(2) Performing of mixing test

A mixing test was performed according to the procedure shown below, using the 8-fold diluted specimen used in section (1), as well as LA-positive plasma samples (specimen Nos. 1 to 5), coagulation factor deficient plasma samples (specimen Nos. 6 and 7) and warfarin-administered patient's plasma samples (specimen Nos. 8 to 10), such as shown in Table 2.

Samples prepared by mixing a test plasma and a normal plasma at ratios of 0, 20, 50 and 100%, were provided, and the coagulation time was measured using the reagent for measuring APTT described in section (1).

Subsequently, the prolongation ratios of coagulation time with respect to a sample containing 0% of a test plasma (containing normal plasma only) were calculated from the respective coagulation times measured, and a polygonal line graph was produced by plotting the data, while using the vertical axis to represent the prolongation ratio of coagulation time and the horizontal axis to represent the proportion of the test plasma.

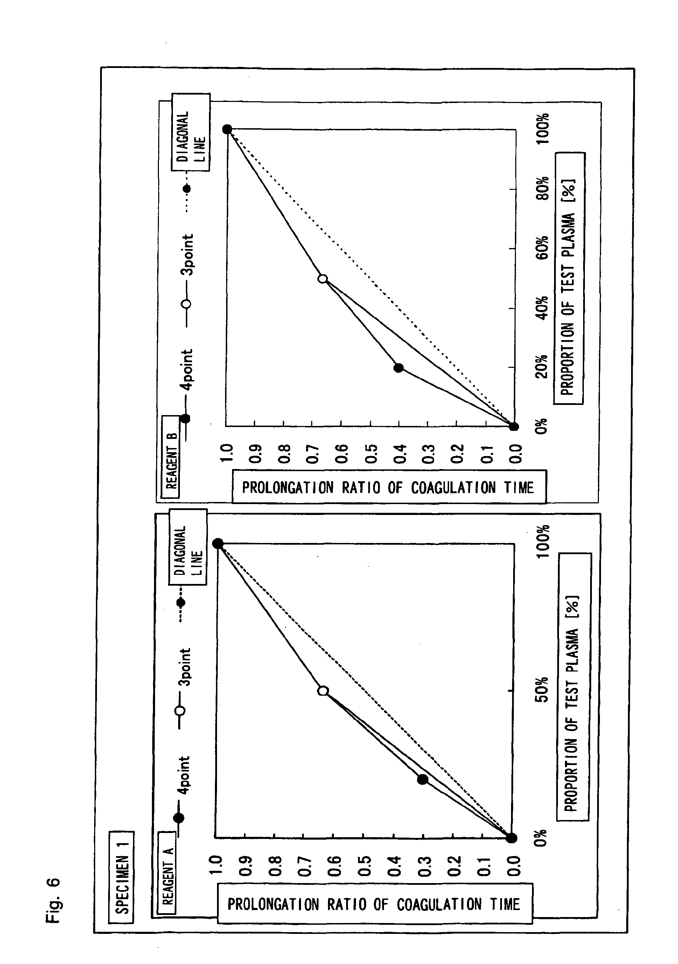

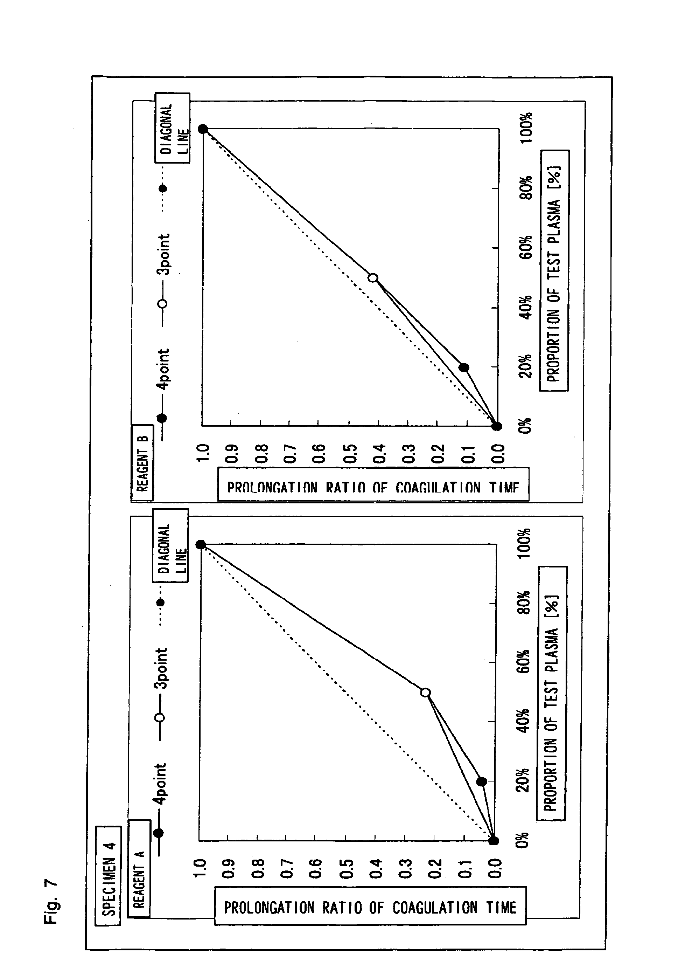

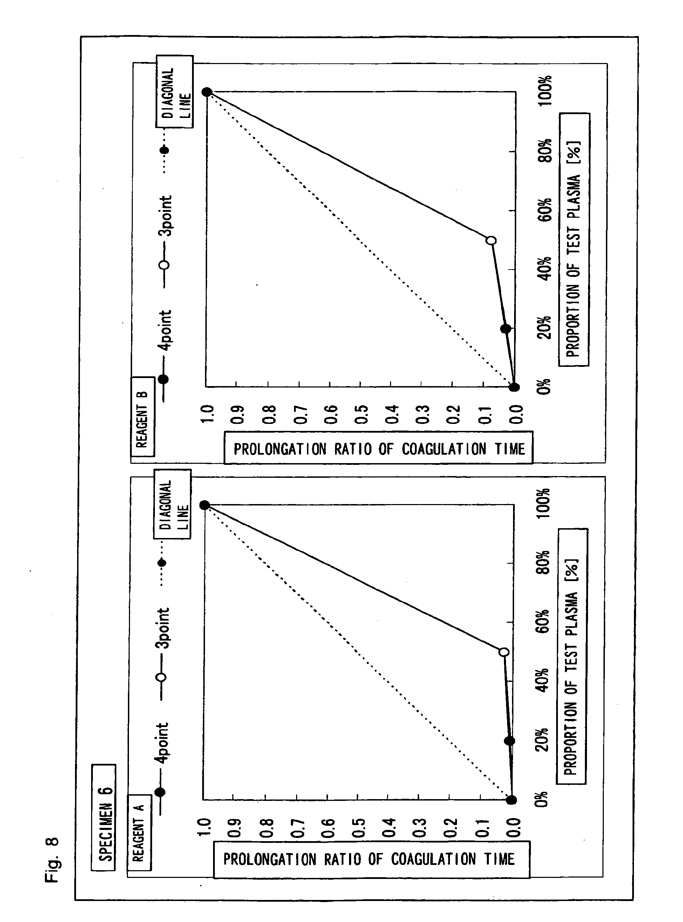

The area of a polygon formed by a diagonal line connecting the prolongation ratio of coagulation time of the sample containing 100% of a test plasma and the prolongation ratio of coagulation time of the sample containing 0% of a test plasma (containing normal plasma only) and by the polygonal line graph (FIGs. 6, 7 and 8) was calculated by subtracting the area of a right-angled triangle having the diagonal as the hypotenuse, from the area under the polygonal line graph. Thus, the proportion of the area of polygon with respect to the area of the right-angled triangle having the diagonal line as the hypotenuse (area ratio (%)) was calculated (Table 2).

The area ratios (%) calculated based on the data of the 8-fold diluted specimens were set as the cut-off values of the respective reagents, and when the area ratio of a sample was equal to or higher than the cut-off value, the sample was determined as the coagulation factor inhibitor type (hereinafter, may be indicated simply as "inhibitor type"), while when the area ratio of a sample was lower than the cut-off value, the sample was determined as the coagulation factor deficient type (hereinafter, may be indicated simply as "factor deficient type") .

This time, the area ratio (%) was calculated by two methods, such as one method in which 3 points were used for the measurement points (proportions of test plasma: 0, 50 and 100%) and one method in which 4 points were used for the measurement points (Proportions of test plasma: 0, 20, 50 and 100%), and the area ratio values thus obtained are indicated in the table as "Invention-3 point" and "Invention-4 point," respectively (Tables 2 and 3).

[Table 2]

| Reagent A | | | | | | | | | |
| --- | --- | --- | --- | --- | --- | --- | --- | --- | --- |
| Specimen No. | Specimen | Proportion of test plasma | | | | Calculation results | | | |
| | | 0% | 20% | 50% | 100% | Invention-3 point | Invention-4 point | Conventional method 1 | Conventional method 2 |
| | 8-Fold diluted sample | 32.2 | 32.1 | 33.6 | 39.9 | -31.8% | -36.1% | | 3.5% |
| 1 | LA-positive patient's plasma 1 | 32.2 | 47.2 | 63.8 | 81.6 | 14.0% | 16.4% | -6.9 | 38.7% |
| 2 | LA-positive patient's plasma 2 | 32.2 | 44.8 | 48.0 | 48.1 | 49.4% | 69.1% | -7.8 | 32.8% |
| 3 | LA-positive patient's plasma 3 | 32.2 | 44.0 | 55.7 | 69.3 | 13.3% | 16.6% | -5.0 | 33.9% |
| 4 | LA-positive patient's plasma 4 | 32.2 | 32.7 | 35.0 | 44.3 | -26.9% | -29.4% | 3.3 | 6.3% |
| 5 | LA-positive patient's plasma 5 | 32.2 | 33.3 | 35.9 | 47.3 | -25.5% | -26.8% | 3.9 | 7.8% |
| 6 | Factor VIII deficient patient's plasma | 32.2 | 32.6 | 34.0 | 100.5 | -47.4% | -47.6% | 32.4 | 1.8% |
| 7 | Factor IX deficient patient's plasma | 32.2 | 31.9 | 32.1 | 79.1 | -50.2% | -50.5% | 23.6 | -0.1% |
| 8 | Warfarin-administered patient's plasma 1 | 32.2 | 31.9 | 31.8 | 39.4 | -55.6% | -56.5% | 4.0 | -1.0% |
| 9 | Warfarin-administered patient's plasma 2 | 32.2 | 32.5 | 33.8 | 43.1 | -35.3% | -36.9% | 3.9 | 3.7% |
| 10 | Warfarin-administered patient's plasma 3 | 32.2 | 31.7 | 31.6 | 43.9 | -55.1% | -56.2% | 6.5 | -1.4% |

Comparative Example 1 (measurement with commercially available LA reagent)

An analysis was performed on the specimens such as described above (specimen Nos. 1 to 5) according to an enclosed document, using commercially available reagents for measuring the LA, LATest "Gradipore" (manufactured by Institute of Medical Biology) and Staclot LA (manufactured by F. Hoffmann-La Roche GmbH).

In regard to the LA Test "Gradipore," the "ratio of coagulation time" (coagulation time of test plasma/coagulation time of normal plasma) was calculated, and a sample having a value of the ratio of 1.3 or higher was determined as LA-positive. In regard to the Staclot LA, the "difference of coagulation time" (coagulation time of test plasma - coagulation time of normal plasma) was calculated, and a sample having a value of the difference of 8 seconds or greater was determined as LA-positive.

Comparative Example 2 (determination of cause of the prolongation of coagulation time according to conventional mixing test method)

The results measured in section (2) were used and applied into the following expressions to perform calculations.

Conventional method 1: (a+c)/2-b

Conventional method 2 (Rosner Index): (b-c)/a

a: Coagulation time of test plasma (seconds)

b: Coagulation time of sample containing 50% of test plasma (seconds)

c: Coagulation time sample containing 0% of test plasma (seconds)

In the conventional method 1, a sample having a value of 0 or less was determined as "inhibitor type," and in the conventional method 2, a sample having a value equal to or greater than the calculated value of the 8-fold diluted specimen used in section (1) of Example 1 as described above, was determined as "inhibitor type."

A comparison of the results of the Example and the Comparative examples are presented in Table 3. Strongly LA-positive specimens (specimen Nos. 1 to 3: positive for both Gradipore and Staclot) were analyzed with the reagent A, and as a result, the specimens were determined as the "inhibitor type, " regardless of whether the determination was made by using the method of the present invention or any one of the conventional methods. However, in the case of weakly positive specimens (specimen Nos. 4 and 5: positive for any one of Gradipore and Staclot), the convention method 1 was not able to detect the "inhibitor type." Furthermore, the same test was performed on LA-negative specimens, and coagulation factor deficient specimens (specimen Nos. 6 and 7) were determined as the "factor deficient type" by all of the calculation methods. However, in the case of warfarin-administered specimens (specimen Nos. 8 to 10), pseudo-positivity was recognized in the conventional method 2 such that the specimen No. 9 was determined as the "inhibitor type," while the methods of the present invention (3 point and 4 point methods) were able to determine the specimen as the "factor deficient type," similarly for both Gradipore and Staclot. From these results, it was found that the methods of the present invention are capable of specifically detecting the "inhibitor type."

[Table 3]

| Reagent A | | | | | | | |
|---|---|---|---|---|---|---|---|
| Specimen No. | | Gradipore | Staclot | Reagent A | | | |
| | | | | Invention-3 point | Invention-4 point | Conventional method 1 | Conventional method 2 |
| | Cut-off value | ≥ 1.30 | ≥ 8.0 | ≥ -31.8% | ≥ -36.1% | ≤ 0 | ≥ 3.5% |
| 1 | LA-positive patient's plasma 1 | 2.18 | 31.3 | 14.0% | 16.4% | -6.9 | 38.7% |
| 2 | LA-positive patient's plasma 2 | 1.70 | 14.7 | 49.4% | 69.1% | -7.8 | 32.8% |
| 3 | LA-positive patient's plasma 3 | 3.05 | 60.3 | 13.3% | 16.6% | -5.0 | 33.9% |
| 4 | LA-positive patient's plasma 4 | 1.25 | 8.3 | -26.9% | -29.4% | 3.3 | 6.3% |
| 5 | LA-positive patient's plasma 5 | 1.33 | 1.2 | -25.5% | -26.8% | 3.9 | 7.8% |
| 6 | Factor VIII deficient patient's plasma | | | -47.4% | -47.6% | 32.4 | 1.8% |
| 7 | Factor IX deficient patient's plasma | | | -50.2% | -50.5% | 23.6 | -0.1% |
| 8 | Warfarin-administered patient's plasma 1 | | | -55.6% | -56.5% | 4.0 | -1.0% |
| 9 | Warfarin-administered patient's plasma 2 | | | -35.3% | -36.9% | 3.9 | 3.7% |
| 10 | Warfarin-administered patient's plasma 3 | | | -55.1% | -56.2% | 6.5 | -1.4% |
| 1 | LA-positive patient's plasma 1 | LA+ | LA+ | Inhibitor type | Inhibitor type | Inhibitor type | Inhibitor type |
| 2 | LA-positive patient's plasma 2 | LA+ | LA+ | Inhibitor type | Inhibitor type | Inhibitor type | Inhibitor type |
| 3 | LA-positive patient's plasma 3 | LA+ | LA+ | Inhibitor type | Inhibitor type | Inhibitor type | Inhibitor type |
| 4 | LA-positive patient's plasma 4 | | LA+ | Inhibitor type | Inhibitor type | | Inhibitor type |
| 5 | LA-positive patient's plasma 5 | LA+ | | Inhibitor type | Inhibitor type | | Inhibitor type |
| 6 | Factor VIII deficient patient's plasma | | | Factor deficient type | Factor deficient type | | |
| 7 | Factor IX deficient patient's plasma | | | Factor deficient type | Factor deficient type | | |

EP 2 290 370 B1

(continued)

| Reagent A | | | | | | | |
|---|---|---|---|---|---|---|---|
| Specimen No. | | Gradipore | Staclot | Reagent A | | | |
| | | | | Invention-3 point | Invention-4 point | Conventional method 1 | Conventional method 2 |
| | Cut-off value | ≥ 1.30 | ≥ 8.0 | ≥ -31.8% | ≥ -36.1% | ≤ 0 | ≥ 3.5% |
| 8 | Warfarin-administered patient's plasma 1 | | | Factor deficient type | Factor deficient type | | |
| 9 | Warfarin-administered patient's plasma 2 | | | Factor deficient type | Factor deficient type | | Inhibitor type |
| 10 | Warfarin-administered patient's plasma 3 | | | Factor deficient type | Factor deficient type | | |

Similarly, the results obtained by an analysis using the reagent B are presented in Tables 4 and 5. The results for the strongly LA-positive specimens (specimen Nos. 1 to 3) were determined as the "inhibitor type," regardless of whether the determination was made by using the method of the present invention or any one of the conventional methods. However, a weakly positive specimen (specimen No. 4), which had been determined as LA-negative with Gradipore, was determined as the "factor deficient type" according to the 3-point method and the conventional method 1. However, when the number of measurement points was increased to 4 points, the same specimen was determined as the "inhibitor type, " and thus, it was found that the degree of accuracy of determination was enhanced.

[Table 4]

| Reagent B | | | | | | | | | |
|---|---|---|---|---|---|---|---|---|---|
| Specimen No. | Specimen | Proportion of test plasma | | | | Calculation results | | | |
| | | 0% | 20% | 50% | 100% | Invention-3 point | Invention-4 point | Conventional method 1 | Conventional method 2 |
| | 8-Fold diluted sample | 26.5 | 27.2 | 30.6 | 35.8 | -5.9% | -11.0% | | 11.5% |
| 1 | LA-positive patient's plasma 1 | 26.5 | 40.9 | 50.4 | 62.6 | 16.2% | 22.9% | -5.9 | 38.2% |
| 2 | LA-positive patient's plasma 2 | 26.5 | 37.7 | 39.3 | 50.3 | 3.8% | 16.6% | -0.9 | 25.4% |
| 3 | LA-positive patient's plasma 3 | 26.5 | 35.5 | 45.6 | 60.2 | 6.7% | 8.7% | -2.3 | 31.7% |
| 4 | LA-positive patient's plasma 4 | 26.5 | 28.0 | 32.2 | 40.1 | -8.1% | -11.0% | 1.1 | 14.2% |
| 5 | LA-positive patient's plasma 5 | 26.5 | 31.5 | 39.0 | 51.0 | 1.0% | 1.0% | -0.3 | 24.5% |
| 6 | Factor VIII deficient patient's plasma | 26.5 | 28.6 | 32.1 | 101. 9 | -42.6% | -42.7% | 32.1 | 5.5% |
| 7 | Factor IX deficient patient's plasma | 26.5 | 28.2 | 30.8 | 90.5 | -43.3% | -43.3% | 27.7 | 4.8% |
| 8 | Warfarin-administered patient's plasma 1 | 26.5 | 27.4 | 29.2 | 41.5 | -32.0% | -32.6% | 4.8 | 6.5% |
| 9 | Warfarin-administered patient's plasma 2 | 26.5 | 27.4 | 29.8 | 39.5 | -24.6% | -26.2% | 3.2 | 8.4% |
| 10 | Warfarin-administered patient's plasma 3 | 26.5 | 27.1 | 28.9 | 46.1 | -37.8% | -38.7% | 7.4 | 5.2% |

[Table 5]

| Reagent B | | | | | | | |
|---|---|---|---|---|---|---|---|
| Specimen No. | | Gradipore | Staclot | Reagent B | | | |
| | | | | Invention-3 point | Invention-4 point | Conventional method 1 | Conventional method 2 |
| | Cut-off value | ≥1.30 | ≥ 8.0 | ≥ -5.9% | ≥ -11.0% | ≤ 0 | ≥ 11.5% |
| 1 | LA-positive patient's plasma 1 | 2.18 | 31.3 | 16.2% | 22.9% | -5.9 | 38.2% |
| 2 | LA-positive patient's plasma 2 | 1.70 | 14.7 | 3.8% | 16.6% | -0.9 | 25.4% |
| 3 | LA-positive patient's plasma 3 | 3.05 | 60.3 | 6.7% | 8.7% | -2.3 | 31.7% |
| 4 | LA-positive patient's plasma 4 | 1.25 | 8.3 | -8.1% | -11.0% | 1.1 | 14.2% |
| 5 | LA-positive patient's plasma 5 | 1.33 | 1.2 | 1.0% | 1.0% | -0.3 | 24.5% |
| 6 | Factor VIII deficient patient's plasma | | | -42.6% | -42.7% | 32.1 | 5.5% |
| 7 | Factor IX deficient patient's plasma | | | -43.3% | -43.3% | 27.7 | 4.8% |
| 8 | Warfarin-administered patient's plasma 1 | | | -32.0% | -32.6% | 4.8 | 6.5% |
| 9 | Warfarin-administered patient's plasma 2 | | | -24.6% | -26.2% | 3.2 | 8.4% |
| 10 | Warfarin-administered patient's plasma 3 | | | -37.8% | -38.7% | 7.4 | 5.2% |
| 1 | LA-positive patient's plasma 1 | LA+ | LA+ | Inhibitor type | Inhibitor type | Inhibitor type | Inhibitor type |
| 2 | LA-positive patient's plasma 2 | LA+ | LA+ | Inhibitor type | Inhibitor type | Inhibitor type | Inhibitor type |
| 3 | LA-positive patient's plasma 3 | LA+ | LA+ | Inhibitor type | Inhibitor type | Inhibitor type | Inhibitor type |
| 4 | LA-positive patient's plasma 4 | | LA+ | Factor deficient type | Inhibitor type | | Inhibitor type |
| 5 | LA-positive patient's plasma 5 | LA+ | | Inhibitor type | Inhibitor type | | Inhibitor type |
| 6 | Factor VIII deficient patient's plasma | | | Factor deficient type | Factor deficient type | | |
| 7 | Factor IX deficient patient's plasma | | | Factor deficient type | Factor deficient type | | |

| Reagent B | | | | | | |
|---|---|---|---|---|---|---|
| Specimen No. | | Gradipore | Staclot | Reagent B | | | |
| | | | | Invention-3 point | Invention-4 point | Conventional method 1 | Conventional method 2 |
| | Cut-off value | ≥1.30 | ≥ 8.0 | ≥ -5.9% | ≥ -11.0% | ≤ 0 | ≥ 11.5% |
| 8 | Warfarin-administered patient's plasma 1 | | | Factor deficient type | Factor deficient type | | |
| 9 | Warfarin-administered patient's plasma 2 | | | Factor deficient type | Factor deficient type | | |
| 10 | Warfarin-administered patient's plasma 3 | | | Factor deficient type | Factor deficient type | | |

**Claims**

1. A method of determining a cause of prolongation of blood coagulation time in test plasma, the method comprising:

    (1) measuring blood coagulation times of samples including (a) the test plasma only, (b) normal plasma only, and (c) the test plasma and the normal plasma mixed at least at a mixing ratio;
    (2) drawing a polygonal line graph by plotting the measurement results of the samples (a), (b) and (c), with a vertical axis representing the blood coagulation time or a prolongation ratio of blood coagulation time and a horizontal axis representing the mixing ratio or mixing proportion of the test plasma or the normal plasma, and thereby determining an area A under the polygonal line and an area B under a line segment that connects the plotted measurement results of the samples (a) and (b);
    (3) calculating a ratio of the area (A-B) obtained by subtracting the area B from the area A, with respect to the area B, ((A-B)/(B)): area ratio X;
    (4) performing the steps of (1) and (2) for a coagulation factor inhibitor-positive plasma, and thereby determining in advance a standard area ratio Y; and
    (5) comparing the area ratio X and the standard area ratio Y, and determining the test plasma as a coagulation factor inhibitor type in the case where $Y \leq X$, or as a coagulation factor deficient type in the case where $Y > X$.

2. The method of claim 1, wherein the standard area ratio Y for the coagulation factor inhibitor-positive plasma is a value determined with respect to a coagulation factor inhibitor-positive plasma exhibiting a blood coagulation time that is larger than the upper limit of a standard range which has been obtained by statistically processing the blood coagulation time of a plurality of healthy persons' plasmas, and is not greater than a value of the upper limit + 50%.

3. The method of claim 2, wherein the standard range for the blood coagulation time of healthy persons' plasmas was obtained by statistically processing the blood coagulation time of a plurality of healthy persons' plasmas.

4. The method of claim 1, wherein when the vertical axis is used to represent the blood coagulation time in (2) of claim 1, values obtained by subtracting a constant number therefrom are used for the area A and the area B.

5. The method of claim 4, wherein the constant number is obtained by drawing an auxiliary line parallel to the horizontal axis and through the plotted point of the blood coagulation time of the normal plasma drawn in the polygonal line graph, and calculating the area under the auxiliary line.

6. The method of any one of claims 1 to 5, wherein the blood coagulation time is at least one selected from PT (Prothrombin time), APTT (activated partial thromboplastin time), dPT (dilute PT), dAPTT (dilute APTT), KCT (kaolin coagulation time), and dRWT (dilute Russell's viper venom time).

7. The method of any one of claims 1 to 6, wherein the coagulation factor inhibitor is at least one selected from Lupus anticoagulant (LA), a factor VIII inhibitor, a factor IX inhibitor, and a factor V inhibitor.

**Patentansprüche**

1. Verfahren zur Bestimmung einer Ursache für eine Verlängerung der Blutkoagulationszeit in Testplasma, wobei das Verfahren umfasst:

    (1) Messung von Blutkoagulationszeiten von Proben einschließlich (a) das Testplasma allein, (b) normales Plasma allein und (c) das Testplasma und das normale Plasma gemischt in mindestens einem Mischungsverhältnis;
    (2) Zeichnen eines polygonalen Liniendiagramms durch Plotten der Messergebnisse der Proben (a), (b) und (c) mit einer vertikalen Achse, die für die Blutkoagulationszeit oder ein Verlängerungsverhältnis der Blutkoagulationszeiten steht, und einer horizontalen Achse, die für das Mischungsverhältnis oder den Mischungsanteil des Testplasmas oder des normalen Plasmas steht, und dadurch Bestimmung einer Fläche A unter dem polygonalen Liniendiagramm und einer Fläche B unter einem Kurvensegment, das die geplotteten Messergebnisse der Proben (a) und (b) verbindet;
    (3) Berechnung des Verhältnisses der Flächen (A-B), erhalten durch Subtraktion der Fläche B von der Fläche A im Hinblick auf die Fläche B ((A-B) / (B)) : Flächenverhältnis X);
    (4) Durchführung der Stufen (1) und (2) für ein Koagulationsfaktor-Inhibitor-positives Plasma und dadurch Bestimmung eines Standardflächenverhältnisses Y im voraus; und

(5) Vergleich des Flächenverhältnisses X und des Standardflächenverhältnisses Y sowie Bestimmung des Testplasmas als vom Koagulationsfaktor-Inhibitor-Typ für den Fall, dass $Y \leq X$, oder als Koagulationsfaktor-defizienter Typ für den Fall, dass $Y > X$.

2. Das Verfahren nach Anspruch 1, wobei das Standardflächenverhältnis Y für das Koagulationsfaktorinhibitor-positive Plasma ein Wert ist, der bestimmt wird im Hinblick auf ein Koagulationsfaktorinhibitor-positives Plasma, das eine Blutkoagulationszeit aufweist, die größer ist als die Obergrenze eines Standardbereichs, der erhalten wurde durch statistisches Verarbeiten der Blutkoagulationszeit von Plasma von gesunden Personen und die nicht höher ist als ein Wert der Obergrenze + 50%.

3. Das Verfahren nach Anspruch 2, wobei der Standardbereich für die Blutkoagulationszeit von Plasma von gesunden Personen durch statistische Verarbeitung der Blutkoagulationszeiten einer Mehrzahl von Plasmen von gesunden Personen erhalten wurde.

4. Das Verfahren nach Anspruch 1, wobei dann, wenn die vertikale Achse verwendet wird, um die Blutkoagulationszeit (2) von Anspruch 1 darzustellen, Werte, die durch Subtrahieren einer konstanten Zahl davon erhalten werden, für die Flächen A und B verwendet werden.

5. Das Verfahren nach Anspruch 4, wobei die konstante Zahl erhalten wird durch Zeichnung einer Hilfslinie parallel zur horizontalen Achse und durch den geplotteten Punkt der Blutkoagulationszeit des normalen Plasmas, in den Polygonkurvenzug die gezeichnet wird, sowie Berechnung der Fläche unter der Hilfslinie.

6. Das Verfahren nach einem der Ansprüche 1 bis 5, wobei die Blutkoagulationszeit ist mindestens eine, ausgewählt aus PT (Prothrombinzeit), APTT (aktivierte partielle Thromboplastinzeit), dPT (verdünnte PT), dAPTT (verdünnte APTT), KCT (Kaolinkoagulationszeit) und dRVVT (verdünnte Russell's Schlangenvenom-Zeit).

7. Das Verfahren nach einem der Ansprüche 1 bis 6, wobei der Koagulationsfaktor-Inhibitor mindestens einer ist, ausgewählt aus Lupus-Antikoagulanz (LA), einem Faktor VIII Inhibitor, einem Faktor IX Inhibitor und einem Faktor V Inhibitor.


**Revendications**

1. Procédé pour déterminer une cause de prolongation du temps de coagulation du sang dans un plasma de test, lequel procédé comprend :

(1) la mesure de temps de coagulation du sang d'échantillons comprenant (a) le plasma de test uniquement, (b) du plasma normal uniquement, et (c) le plasma de test et le plasma normal mélangés en au moins un rapport de mélange ;

(2) le tracé d'un graphique linéaire polygonal par report des résultats de mesure des échantillons (a), (b) et (c), l'axe vertical représentant le temps de coagulation du sang ou un rapport de prolongation du temps de coagulation du sang, et l'axe horizontal représentant le rapport de mélange ou la proportion de mélange du plasma de test ou du plasma normal, et ainsi la détermination d'une zone A sous la ligne polygonale et d'une zone B sous un segment de ligne qui connecte les résultats de mesure reportés des échantillons (a) et (b) ;

(3) le calcul d'un rapport de zones (A-B) obtenu par soustraction de la zone B de la zone A, par rapport à la zone B, ((A-B)/(B)) : rapport de zones X) ;

(4) la mise en oeuvre des étapes (1) et (2) pour un plasma positif à un inhibiteur de facteur de coagulation, et ainsi la détermination à l'avance d'un rapport de zones standard Y ; et

(5) la comparaison du rapport de zones X et du rapport de zones standard Y, et la détermination du plasma de test en tant que type d'inhibiteur de facteur de coagulation dans le cas où $Y \leq X$, ou en tant que type déficient en facteur de coagulation dans le cas où $Y > X$.

2. Procédé selon la revendication 1, dans lequel le rapport de zones standard Y pour le plasma positif à un inhibiteur de facteur de coagulation est une valeur déterminée par rapport à un plasma positif à un inhibiteur de facteur de coagulation présentant un temps de coagulation du sang qui est supérieur à la limite supérieure d'une plage standard qui a été obtenue par traitement statistique du temps de coagulation du sang de plusieurs plasmas de personnes en bonne santé, et n'est pas supérieure à la valeur de la limite supérieure + 50 %.

3. Procédé selon la revendication 2, dans lequel la plage standard pour le temps de coagulation du sang de plasmas de personnes en bonne santé a été obtenue par traitement statistique du temps de coagulation du sang de plusieurs plasmas de personnes en bonne santé.

4. Procédé selon la revendication 1, dans lequel l'axe vertical est utilisé pour représenter le temps de coagulation du sang dans (2) de la revendication 1, et les valeurs obtenues par soustraction d'un nombre constant de celui-ci étant utilisées pour la zone A et la zone B.

5. Procédé selon la revendication 4, dans lequel le nombre constant est obtenu par le tracé d'une ligne auxiliaire parallèle à l'axe horizontal et passant par le point reporté du temps de coagulation du sang du plasma normal tracé dans le graphique linéaire polygonal, et calcul de la zone sous la ligne auxiliaire.

6. Procédé selon l'une quelconque des revendications 1 à 5, dans lequel le temps de coagulation du sang est au moins un temps choisi parmi le PT (temps de prothrombine), l'APTT (temps de thromboplastine partielle activée), le dPT (PT dilué), le dAPTT (APTT dilué), le KCT (temps de coagulation du kaolin), et le dRVVT (temps du venin de vipère de Russell dilué).

7. Procédé selon l'une quelconque des revendications 1 à 6, dans lequel l'inhibiteur de facteur de coagulation est au moins un inhibiteur choisi parmi l'anticoagulant lupique (LA), un inhibiteur de facteur VIII, un inhibiteur de facteur IX, et un inhibiteur de facteur V.

Fig. 1

Fig. 2

Fig. 3

Fig. 4

Fig. 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Kensa To Gijutsu,* August 2006, vol. 34 (8), 735-742
- Rinsho Kensaho Teiyo. 443
- *Thrombo. Haemost.,* 1987, vol. 57 (2), 144-147